# FASCICULE DE BREVET EUROPEEN

(11) **EP 3 852 866 B1**
(45) Date de publication et mention de la délivrance du brevet: **17.05.2023**
(21) Numéro de dépôt: 19786645.2
(22) Date de dépôt: 10.09.2019
(51) Int. Cl.: A61N 1/16, A61N 1/20, A61N 1/24

(54) **DISPOSITIF DE STIMULATION ÉNERGÉTIQUE POURVU D'UN TRANSMETTEUR DE STIMULATION**
VORRICHTUNG ZUR ENERGIESTIMULATION MIT EINEM STIMULATIONSGEBER
ENERGY STIMULATION DEVICE PROVIDED WITH A STIMULATION TRANSMITTER

(30) Priorité: 20.09.2018 FR 1858528
(43) Date de publication de la demande: 28.07.2021
(73) Titulaire: Bricot, Bernard, 83150 Bandol (FR)
(72) Inventeur: Bricot, Bernard, 83150 Bandol (FR)
(74) Mandataire: Weber, Etienne Nicolas
(86) Numéro de dépôt international: PCT/FR2019/052083
(87) Numéro de publication internationale: WO 2020/058599

(56) Documents cités:
- EP-A1- 0 145 673
- FR-A1- 2 642 654
- FR-A1- 2 873 917
- GB-A- 2 154 140

## Description

### Domaine technique

La présente Invention concerne un dispositif de stimulation énergétique.

Le terme "énergétique" utilisé dans la suite du texte, s'entend dans son acception usuelle dans les domaines de la médecine énergétique, de l'acuponcture, ou des pratiques énergétiques appliquées au corps humain ou aux plantes.

L'invention trouve des applications thérapeutiques notamment pour le traitement de désordres posturaux et de pathologies ou de symptômes directement ou Indirectement induits par un désordre postural.

En outre, le dispositif est utilisable comme Instrument de bio-stimulation pour le traitement de douleurs induites ou non par un désordre postural. Il permet ainsi de traiter des rachialgies, des cervlcalgies, des lombalgies, des dorsalgies, mais aussi des déchirures musculaires, des entorses ou des points douloureux du type "trigger point". Le dispositif peut également être mis en œuvre pour le traitement de troubles de la statique, telles que des scolioses, des cyphoses ou des bascules du bassin. Il peut encore être mis en oeuvre pour le traitement spécifique de céphalées, de névralgies, de scapulalgies et de myalgies.

Indépendamment du domaine médical ou thérapeutique, l'invention trouve aussi des applications pour favoriser la croissance ou la maturation de plantes et de fruits, et en particulier dans le domaine vitivinicole pour la croissance de la vigne, l'élevage de vin, et la conservation du vin.

De manière plus générale, l'invention trouve des applications pour la bio-stimulation humaine ou animale, ainsi que pour la bio-stimulation dans le domaine agricole ou agmalimentaire.

### Etat de la technique antérieure

L'invention constitue un perfectionnement d'un dispositif de stimulation connu et décrit, par exemple, dans les brevets suivants :
EP0145673,
EP 1 218 058, FR 2798843 et US6773391. Un tel dispositif est encore désigné par dispositif à champ électro-galvanique.

Le dispositif comprend pour l'essentiel un générateur de stimulation, ou source de stimulation sous la forme d'une plaquette plane comprenant deux métaux différents en contact mutuel et formant un couple galvanique.

Le générateur peut être utilisé tel quel ou être intégré dans un vêtement, un support de contention, ou une semelle pour le maintenir au voisinage d'une partie du corps douloureuse ou à traiter.

### Exposé de l'invention

L'invention procède d'une recherche d'amélioration des performances du générateur de stimulation connu et d'une recherche de diversification de ses applications.

Un but de l'invention est en particulier de proposer un dispositif de stimulation amélioré, plus ergonomique et plus efficace dans la bio-stimulation du corps humain et animal.

Un autre but de l'invention est de faciliter l'utilisation d'un dispositif de bio-stimulation à visée thérapeutique tout en affranchissant l'utilisateur de la contrainte de porter un vêtement spécifique, des semelles ou tout autre accessoire maintien du dispositif.

Un but de l'invention est encore de proposer un dispositif de stimulation adapté à des applications non-médicales ou non-thérapeutiques, par exemple pour la croissance de végétaux ou pour la fabrication et la conservation de vin.

Pour atteindre ces buts, l'invention propose un dispositif de stimulation énergétique comprenant au moins un générateur de stimulation pourvu d'au moins un premier corps métallique et d'au moins un deuxième corps métallique, en contact électrique et mécanique avec le premier corps métallique, le premier corps métallique et le deuxième corps métallique comprenant des métaux différents.

Conformément à l'invention, le dispositif comprend en outre au moins un transmetteur de stimulation, le transmetteur de stimulation comprenant un support rigide distinct du générateur de stimulation, et le générateur de stimulation étant fixé sur le support rigide.

On considère que le premier corps métallique est en contact mécanique avec le deuxième corps métallique lorsqu'ils se touchent mutuellement. Il s'agit d'un contact mécanique direct

On considère que le générateur de stimulation est fixé sur le support rigide lorsqu'il est rigidement solidaire du support rigide.

Le générateur de stimulation est utilisé comme source de stimulation. Il est assimilé à un résonateur fréquentiel sur la base de cycles de charge et de décharge énergétique.

On considère que le premier corps métallique et le deuxième corps métallique du générateur de stimulation comprennent des métaux différents lorsqu'ils sont réalisés dans des métaux différents c'est-à-dire de numéro atomique différent, mais aussi lorsqu'ils sont réalisés à partir d'alliages de métaux différents.

En d'autres termes, le premier corps métallique et le deuxième corps métallique forment un couple galvanique.

On peut utiliser de préférence des métaux présentant des potentiels électrochimiques éloignés, de manière à former un couple galvanique avec une différence de potentiel élevée.

Il convient toutefois de noter que des métaux avec des potentiels éloignés, peuvent être sujets à un phénomène de corrosion galvanique en raison de leur mise en contact. Ce phénomène peut conduire à un épuisement du générateur de stimulation, et une diminution des effets du dispositif de stimulation dans le temps. Au besoin, un remplacement du générateur de stimulation peut être opéré.

De manière avantageuse, le premier corps métallique peut être réalisé en cuivre et le deuxième corps métallique peut être réalisé en zinc, de manière à ailier une bonne efficacité et une bonne pérennité du dispositif de stimulation.

Le générateur de stimulation peut comporter un seul ou plusieurs couples galvaniques, c'est-à-dire un ou plusieurs premiers corps métalliques associés à un ou plusieurs deuxièmes corps métalliques. La référence au singulier au premier corps métallique et au deuxième corps métallique ne préjuge pas ainsi du nombre de couples galvaniques constituant le générateur de stimulation.

De préférence le générateur de stimulation peut se présenter sous la forme d'une plaquette plate avec une face principale sensiblement plane tournée vers le support rigide. La mise en contact du générateur de stimulation et le support rigide du transmetteur de stimulation par la face principale permet de garantir un bon couplage énergétique. La fixation peut avoir lieu simplement par collage, par sertissage, ou par complémentarité de forme, par exemple.

On considère que la face principale est une face de plus grande surface. Le générateur de stimulation lorsqu'il se présente sous la forme d'une pastille, peut présenter deux faces opposées identiques, ou pour le moins de même surface. Dans ce cas, l'une des faces est considérée comme la face principale et est tournée vers le support rigide.

Le générateur de stimulation est de préférence fixé sur le support rigide par l'intermédiaire de sa face tournée vers le support rigide. Par exemple, la face principale tournée vers le support rigide peut être collée au support rigide.

Selon d'autres possibilités encore, le générateur de stimulation peut être serti dans le support rigide ou noyé dans la matière du support rigide.

En ce qui concerne la conformation du générateur de stimulation on peut se reporter, au document FR 2798843, par exemple. Selon un mode d'exécution possible, le premier corps métallique et le deuxième corps métallique peuvent être des pastilles superposées, présentant des faces principales en contact. La mise en contact des faces principales, c'est à dire les faces de plus grande surface permet d'optimiser le contact électrique et galvanique. Selon une autre possibilité, le premier corps métallique et le deuxième corps métalliques peuvent également être concentriques.

De manière générale, le premier corps métallique et le deuxième corps métallique sont agencés de manière à être en contact électrique mutuel afin de former un couple galvanique.

L'association du générateur de stimulation avec un transmetteur de stimulation, et en particulier un transmetteur de stimulation comprenant un support rigide, permet d'améliorer l'effet de stimulation par ractivation du transmetteur. L'amélioration peut s'avérer assez nette notamment dans une configuration où le dispositif de l'invention est configuré sous la forme d'un applicateur de stimulation tenu à la main.

On considère que le transmetteur de stimulation comprend un support rigide lorsque tout ou partie du transmetteur est rigide. En particulier le transmetteur de stimulation peut comporter le support rigide, rigidement solidaire du générateur de stimulation, et également une partie non rigide. La partie non rigide peut être flexible ou molle, solide ou liquide, ou sous forme de gel.

A titre d'illustration la partie non rigide peut être un coussin ou un tapis de sol dans lequel le support rigide et le générateur de stimulation sont intégrés.

On considère que le support est "rigide" lorsqu'il n'est pas possible de fléchir le support de manière visible, sans outil et par la seule force humaine. Le caractère rigide dudit support rigide peut lui être conféré par un choix de matière mais aussi par un choix dimensionnel et en particulier de l'épaisseur.

En particulier le support rigide du transmetteur de stimulation peut être réalisé en un matériau choisi parmi : le verre, la pierre, le bois, un minéral et une céramique. D'autres matériaux rigides tels qu'un matériau composite ou une résine peuvent également être retenus.

Dans une forme de réalisation particulièrement avantageuse du dispositif, le transmetteur peut être un cristal de roche, le cristal de roche constituant le support rigide. L'utilisation d'un cristal de roche comme transmetteur de stimulation permet d'obtenir une stimulation particulièrement efficace notamment dans l'axe du cristal c'est-à-dire selon un axe parallèle aux facettes latérales naturelles du cristal de roche. Une stimulation du pouls peut être obtenue à plusieurs mètres dans l'axe du cristal.

Selon encore une autre possibilité, le support rigide, formant le transmetteur de stimulation, peut être une pierre polie du type utilisée en lithothérapie.

De manière plus générale, le support rigide peut, de préférence, être en un matériau Isolant électrique.

Par ailleurs, le support rigide peut présenter de préférence des dimensions et/ou une masse, supérieures à celles du générateur de stimulation.

A titre d'exemple, pour un dispositif de stimulation sous la forme d'un applicateur manuel peut présenter un ou plusieurs générateurs de stimulation, respectivement sous la forme d'une pastille de 1 à 5 cm de diamètre, et une épaisseur de l'ordre du millimètre ou de quelques millimètres. Le support rigide, par exemple un cristal de roche, peut présenter une longueur de 10 à 20 cm et un diamètre de 4 à 5 cm, voire davantage.

Le transmetteur de stimulation peut présenter une forme adaptée à l'usage et la destination du dispositif de stimulation.

Par exemple, pour un usage du dispositif comme un applicateur manuel, le transmetteur peut présenter au moins l'un parmi une partie présentant une face plane et polie, et une partie formant une pointe.

Une face polie peut être mise à profit pour effectuer un massage d'une zone du corps devant être stimulée. En revanche, une partie formant une pointe facilite la transmission de la stimulation vers des points d'acuponcture, des points d'acupressure, des chakras ou simplement vers des zones douloureuses.

Une ou plusieurs applications du dispositif de stimulation sur les parties du corps à traiter ou sur les parties douloureuses, respectivement pendant une durée de quelques minutes, permettent d'apporter des corrections posturales.

D'autres configurations du dispositif de stimulation, et du transmetteur de stimulation, peuvent être envisagées.

Le transmetteur de stimulation peut notamment être configuré sous la forme de l'un des articles suivants:
- un contenant alimentaire,
- un siège,
- une partie de siège,
- une partie de lit,
- un coussin,
- un tapis,
- une prothèse,
- une chaussure,
- un contenant de culture.

Dans le cas d'une chaussure, le support rigide du transmetteur de stimulation peut constituer, par exemple, un talon de chaussure.

Dans le cas d'une prothèse, le support rigide peut constituer, par exemple, une armature de la prothèse.

Un siège, par exemple une chaise ou un fauteuil, ou une partie de siège, un dossier ou une assise, peuvent également constituer le transmetteur de stimulation pour des séances de bio-stimulation en position assise. Il en va de même pour un lit, ou une partie de lit pour une bio-stimulation en position allongée.

Un dispositif de stimulation sous la forme d'une chaise ou d'un fauteuil permet de renouveler une stimulation à chaque fois qu'un utilisateur prend place sur la chaise et permet ainsi de procurer un traitement prolongé. Dans le cas d'un coussin ou d'un tapis, le support rigide et le générateur de stimulation peuvent être intégrée dans une partie ne compromettant pas le confort d'utilisation.

Le transmetteur de stimulation peut également être un contenant alimentaire et en particulier une bouteille.

L'utilisation d'un transmetteur de stimulation sous la forme d'un contenant alimentaire et/ou d'une bouteille permet, par exemple, une utilisation pour une meilleure conservation et une meilleure maturation du vin.

Enfin, le transmetteur de stimulation peut se présenter sous la forme d'un contenant de culture, par exemple un bac de culture ou plus simplement un pot pour fleurs.

On entend par contenant de culture un contenant susceptible de recevoir un substrat pour la culture de plantes, de cellules animales ou de cellules végétales. Le substrat de culture peut être considéré comme faisant partie du transmetteur, dont la partie rigide est, par ailleurs, constituée par une paroi du contenant de culture.

Le dispositif de l'invention configuré sous la forme d'un contenant de culture permet en particulier de stimuler la germination et la croissance de plantes vertes, de plantes potagères ou de plantes d'agrément. Il s'agit dans ce cas de plantes cultivées dans un substrat de culture reçu dans le contenant de culture.

D'autres caractéristiques et avantages de l'invention ressortent de la description qui suit, en référence aux figures des dessins. Cette description est donnée à titre purement illustratif et non limitatif.

### Brève description des figures

La figure 1 est une représentation schématique d'un dispositif de stimulation conforme à l'invention.
La figure 2 est une coupe schématique d'un générateur de stimulation utilisable dans un dispositif de stimulation conforme à l'invention.
La figure 3 est une coupe schématique d'un autre générateur de stimulation utilisable dans un dispositif de stimulation conforme à l'invention.
La figure 4 est une représentation schématique d'un dispositif de stimulation conforme à l'invention configuré sous la forme d'un siège.
La figure 5 est une représentation schématique d'un dispositif de stimulation conforme à l'invention configuré sous la forme d'un coussin ou d'un tapis de sol.
La figure 6 est une coupe schématique selon un plan de symétrie d'un dispositif de stimulation conforme à l'invention configuré sous la forme d'un contenant alimentaire, et en particulier d'une bouteille.
La figure 7 est une coupe schématique selon un plan de symétrie d'un dispositif de stimulation conforme à l'invention configuré sous la forme d'un contenant de culture.
La figure 8 est une représentation schématique d'un dispositif de stimulation conforme à l'invention configuré sous la forme d'une chaussure.

Les figures sont exécutées en échelle libre.

### Description détaillée de modes de mise en œuvre de l'invention

Dans la description qui suit, des parties identiques, similaires ou équivalentes, des différentes figures, sont Indiquées avec les mêmes signes de référence de manière à faciliter le report d'une figure à l'autre.

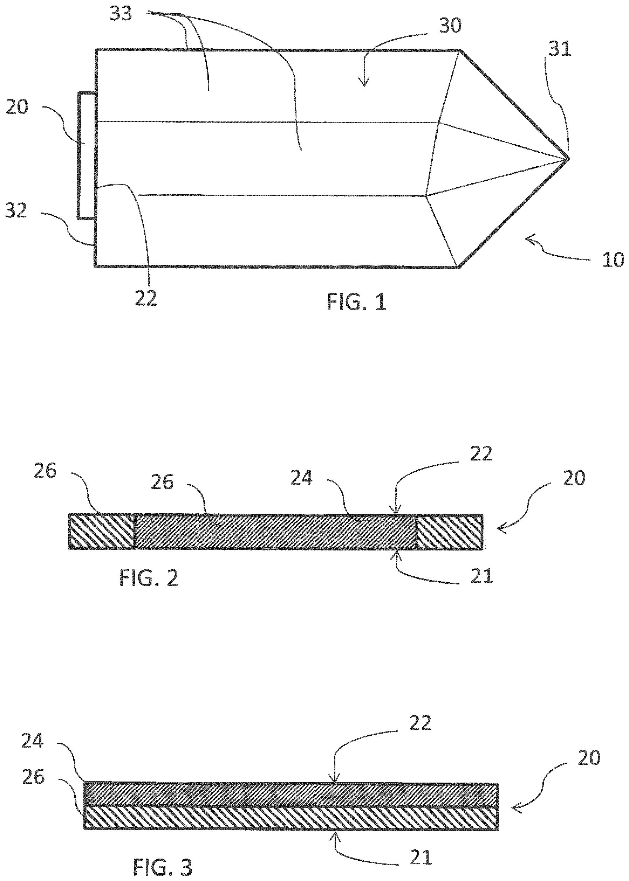

La figure 1 montre un dispositif de stimulation 10 conforme à l'invention comprenant un générateur de stimulation 20, ou source, et un transmetteur de stimulation 30.

Le générateur de stimulation 20 se présente sous la forme d'une pastille bimétallique plate. Il présente une face principale 12, plane, en contact avec le transmetteur de stimulation 30.

Le transmetteur de stimulation 30 est un bloc de cristal de roche. Il présente une extrémité avant formant une pointe 31, des faces latérales 33 planes et lisses, et une extrémité arrière plane qui constitue un support rigide 32 recevant le générateur de stimulation 20.

La face principale 22 du générateur de stimulation est maintenue rigidement sur le support rigide 32, par exemple, par collage ou par sertissage.

Un dispositif de stimulation comparable à celui de la figure 1 peut être utilisé, par exemple, en appliquant le transmetteur de stimulation 30 contre le corps ou contre un vêtement d'un patient dans une zone douloureuse. Selon une autre possibilité, la pointe 31 du transmetteur de stimulation 30 peut être orientée vers un point d'acuponcture et/ou être mise en contact avec le point d'acuponcture.

Les dimensions du transmetteur de stimulation peuvent être variables selon le bloc de cristal de roche utilisé. Elles sont de préférence adaptées à une prise en main aisée.

Des modèles plus économiques d'un dispositif de stimulation conforme à la figure 1 peuvent être réalisés en remplaçant le cristal de roche par un bloc de verre, ou de pierre polie, par exemple.

La figure 2 montre en coupe une possibilité de réalisation du générateur de stimulation 20 de la figure 1.

Dans ce mode de réalisation le générateur de stimulation est formé par un sertissage concentrique de deux corps métalliques 24, 28. Un premier corps métallique 24, par exemple en cuivre, se présente sous la forme d'une pastille. Un deuxième corps métallique 26, par exemple en zinc, se présente sous la forme d'un anneau recevant, en son centre, la pastille du premier corps métallique. Les deux corps métalliques présentent la même épaisseur de manière à leur conférer des faces principales 21, 22 planes. Lorsque le générateur de stimulation 20 de la figure 2 est associé à un transmetteur de stimulation, l'une des faces principales 21, 22 est appliquée sur le support rigide du transmetteur. Elle peut en particulier y être collée.

La figure 3 montre encore une autre possibilité de réalisation dans laquelle le générateur de stimulation est constitué de deux pastilles métalliques 24, 26 en contact mécanique et électrique mutuel par des faces principales. Les deux pastilles sont, par exemple, une pastille de cuivre et une pastille de zinc ou une pastille de laiton et une pastille d'acier.

Les pastilles métalliques peuvent être fixées l'une à l'autre par sertissage ou par emboîtement, par exemple.

Lorsque le générateur de stimulation 20 de la figure 3 est associé à un transmetteur de stimulation, l'une des pastilles est appliquée sur le support rigide du transmetteur par sa face principale libre 21, 22.

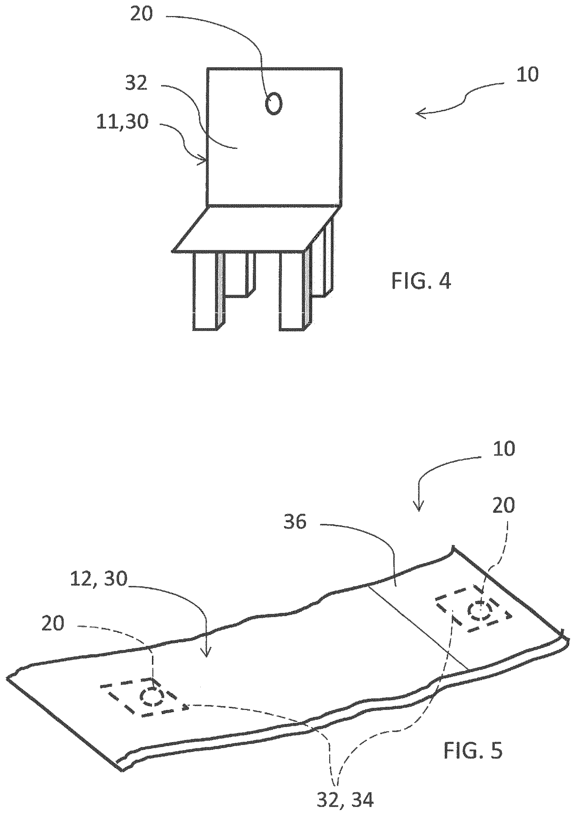

La figure 4 illustre une mise en œuvre de l'invention dans laquelle le dispositif de stimulation 10 et en particulier le transmetteur de stimulation 30 est un siège 11.

Il s'agit plus particulièrement d'une chaise dont le dossier constitue le support rigide 32 du transmetteur de stimulation. Le dossier est, par exemple, un dossier en bois, ou en matériau composite. Il reçoit le générateur de stimulation 20. Le générateur peut être simplement collé contre le dossier, noyé dans un matériau constituant le dossier ou serti dans un logement pratiqué dans le dossier.

La figure 5 illustre encore une autre possibilité de mise en oeuvre du dispositif de l'invention 10 sous la forme d'un tapis de sol 12.

Le tapis de sol 12 se présente pour l'essentiel sous la forme d'une plaque de mousse, souple, qui constitue le transmetteur de stimulation 30. Le tapis de sol comprend également une partie 38, formant coussin, et constituant également un transmetteur de stimulation.

Conformément à l'invention, l'ensemble du transmetteur n'est pas souple. En effet, des plaques rigides 34, par exemple, des plaques de matière plastique rigide, sont appliquées contre la plaque de mousse ou noyés dans la plaque de mousse. Elles constituent respectivement des supports rigides 32 au sens de l'invention et reçoivent chacune un générateur de stimulation 20.

Les générateurs de stimulation et les supports rigides ne sont pas visible de la face supérieure du tapis 12 et sont ainsi représentés en trait discontinu.

Dans l'exemple de la figure 5, deux générateurs de stimulation 20 sont représentés. Ils sont associés respectivement à deux supports rigides 32, 34. Il convient à cet égard de préciser qu'un nombre plus grand de générateurs de stimulation, et un nombre plus grand de supports rigides peuvent être mis en oeuvre. De même, chaque support rigide peut porter plusieurs générateurs de stimulation.

Un lit, une partie de lit, un matelas, ou un coussin, peuvent être configurés d'une manière analogue à celle du tapis de la figure 5.

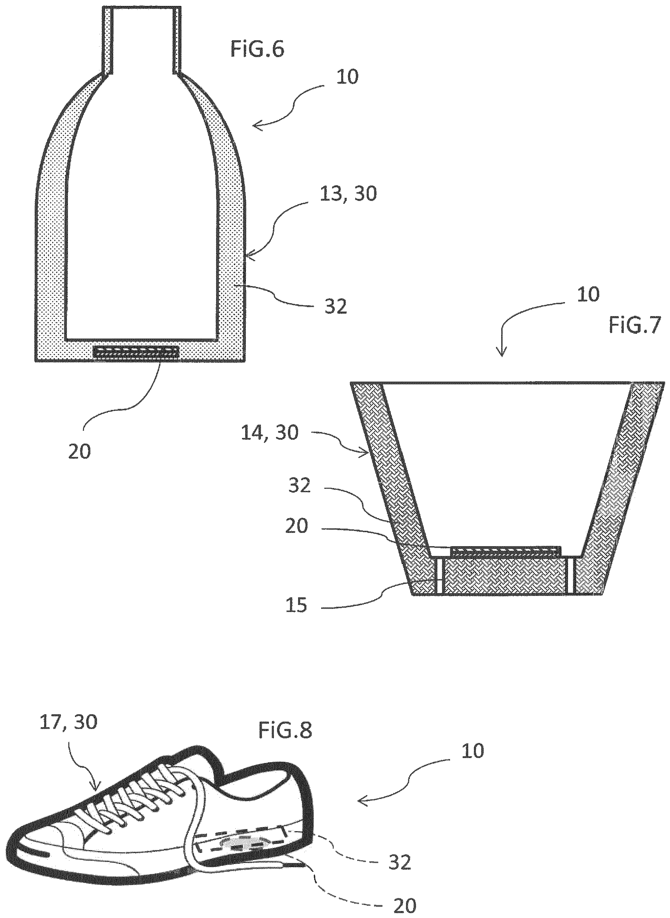

La figure 6 montre une possibilité de mise en œuvre de l'invention dans laquelle le dispositif de stimulation 10, et plus précisément le transmetteur de stimulation 30, est un contenant alimentaire. Il s'agit plus précisément d'une bouteille 13. Elle est représentée en coupe.

La paroi de la bouteille 13 est une paroi rigide, réalisée, par exemple, en verre, et constitue un support rigide 32 au sens de l'invention et également le transmetteur de stimulation 30. Le générateur de stimulation 20 se présente sous la forme d'une pastille en un bimétal collée contre la paroi de la bouteille ou noyée dans la paroi de la bouteille 13. Le générateur de stimulation peut, par exemple, trouver place dans le culot de la bouteille.

Une telle bouteille peut être mise en œuvre pour favoriser la conservation et le vieillissement de vin. D'une manière similaire, un ou plusieurs générateurs de stimulation peuvent être appliqués sur la paroi d'une cuve ou d'une barrique pour favoriser l'élevage de vin.

La figure 7 montre, en coupe un contenant de culture 14, tel qu'un bac de culture ou un pot de fleur qui constitue le transmetteur de stimulation 30 d'un dispositif de stimulation 10 conforme à l'invention.

La paroi du contenant de culture 14, par exemple en terre cuite, constitue le support rigide 32 du transmetteur de stimulation.

En effet un générateur de stimulation 20, bimétal, est collé contre la paroi du contenant de culture.

Dans l'exemple de réalisation représenté, le générateur de stimulation est collé à l'intérieur du contenant de culture 14 sur une paroi formant le fond.

Le générateur peut également être ménagé sur une quelconque autre partie de la paroi, à l'intérieur ou à l'extérieur du contenant de culture 14.

La référence 15 indique des drains pratiqués dans le fond du contenant de culture 14.

La figure 8, montre une réalisation d'un dispositif de stimulation 10, conforme à l'invention sous la forme d'une chaussure 17. La chaussure, et en particulier le talon de la chaussure forme un transmetteur de stimulation 30.

Dans ce mode de réalisation le talon comprend un générateur de stimulation 20 solidaire au besoin d'un insert rigide 32 formant le support rigide.

De manière comparable à la figure 8, un dispositif de stimulation peut également constituer un vêtement, une orthèse ou une prothèse.

## Revendications

1. Dispositif de stimulation énergétique (10) comportant au moins un générateur de stimulation (20) pourvu d'au moins un premier corps métallique (24) et d'au moins un deuxième corps métallique (28) en contact électrique et mécanique avec le premier corps métallique, le premier corps métallique et le deuxième corps métallique comprenant des métaux différents,
le dispositif comporte en outre au moins un transmetteur (30) de stimulation, le transmetteur de stimulation comprenant un support rigide (32) distinct du générateur de stimulation, et le générateur de stimulation (20) étant fixé sur le support rigide (30), le support rigide n'étant pas flexible manuellement sans outil,
**caractérisé en ce que** :
le générateur de stimulation (20) se présente sous la forme d'une plaquette plate avec une face principale (22) sensiblement plane tournée vers le support rigide (32).

2. Dispositif selon la revendication 1, dans lequel le support rigide (32) du transmetteur de stimulation (30) présente des dimensions supérieures à celles du générateur de stimulation (20).

3. Dispositif selon l'une quelconque des revendications 1 à2, dans lequel le support rigide (32) est en un matériau isolant électrique.

4. Dispositif selon le revendication 3, dans lequel le support rigide (32) du transmetteur de stimulation (30) est réalisé en un matériau choisi parmi : le verre, la pierre, le bois, un minéral et une céramique.

5. Dispositif selon l'une quelconque des revendications précédentes, dans lequel le transmetteur de stimulation (30) est un cristal de roche, le cristal de roche constituant ledit support rigide (32).

6. Dispositif selon l'une quelconque des revendications précédentes, dans lequel le premier corps métallique (24) est réalisé on cuivre et le deuxième corps métallique (26) est réalisé en zinc.

7. Dispositif selon l'une quelconque des revendications précédentes, dans lequel le premier corps métallique (24) et le deuxième corps métallique (26) sont des pastilles superposées, présentant des faces principales en contact.

8. Dispositif selon l'une quelconque des revendications 1 à 6, dans lequel le premier corps métallique (24) et le deuxième corps métallique (26) sont concentriques.

9. Dispositif selon l'une quelconque des revendications précédentes, dans lequel le transmetteur de stimulation (30) présente au moins l'un parmi une face plane polie (33) et une partie formant une pointe (31).

10. Dispositif selon l'une quelconque des revendications précédentes, dans lequel le transmetteur de stimulation (30) est configuré sous la forme de l'un des articles suivants :
- un contenant alimentaire (13),
- un siège (11),
- une partie de siège (11),
- une partie de lit,
- une prothèse,
- une chaussure (17),
- un contenant de culture (14).

11. Dispositif selon te revendication 10, dans lequel le transmetteur de simulation (30) est une bouteille (13).

## Patentansprüche

1. Vorrichtung zur energetischen Stimulation (10), die mindestens einen Stimulationsgenerator (20) umfasst, der mit mindestens einem ersten Metallkörper (24) und mindestens einem zweiten Metallkörper (28) versehen ist, welcher sich mit dem ersten Metallkörper in elektrischem und mechanischem Kontakt befindet, wobei der erste Metallkörper und der zweite Metallkörper verschiedene Metalle umfassen,
die Vorrichtung weiter mindestens einen Stimulationsgeber (30) umfasst, wobei der Stimulationsgeber einen starren Träger (32) umfasst, der sich vom Stimulationsgenerator unterscheidet, und wobei der Stimulationsgenerator (20) am starren Träger (30) befestigt ist, wobei der starre Träger von Hand nicht ohne Werkzeug gebogen werden kann,
**dadurch gekennzeichnet, dass**:
der Stimulationsgenerator (20) sich in Form einer flachen Platte mit einer im Wesentlichen planen, dem starren Träger (32) zugewandten Hauptseite (22) zeigt.

2. Vorrichtung nach Anspruch 1, wobei der starre Träger (32) des Stimulationsgebers (30) Abmessungen aufweist, die größer sind als die des Stimulationsgenerators (20).

3. Vorrichtung nach einem der Ansprüche 1 bis 2, wobei der starre Träger (32) aus einem elektrisch isolierenden Material besteht.

4. Vorrichtung nach Anspruch 3, wobei der starre Träger (32) des Stimulationsgebers (30) aus einem Material hergestellt ist, ausgewählt aus: Glas, Stein, Holz, einem Mineral und einer Keramik.

5. Vorrichtung nach einem der vorstehenden Ansprüche, wobei es sich bei dem Stimulationsgeber (30) um einen Bergkristall handelt, wobei der Bergkristall den starren Träger (32) darstellt.

6. Vorrichtung nach einem der vorstehenden Ansprüche, wobei der erste Metallkörper (24) aus Kupfer hergestellt ist, und der zweite Metallkörper (26) aus Zink hergestellt ist.

7. Vorrichtung nach einem der vorstehenden Ansprüche, wobei es sich bei dem ersten Metallkörper (24) und dem zweiten Metallkörper (26) um übereinanderliegende Plättchen handelt, die in Kontakt befindliche Hauptseiten aufweisen.

8. Vorrichtung nach einem der Ansprüche 1 bis 6, wobei der erste Metallkörper (24) und der zweite Metallkörper (26) konzentrisch sind.

9. Vorrichtung nach einem der vorstehenden Ansprüche, wobei der Stimulationsgeber (30) mindestens eines aufweist aus einer polierten Planfläche (33) und einem Teil, der eine Spitze (31) bildet.

10. Vorrichtung nach einem der vorstehenden Ansprüche, wobei der Stimulationsgeber (30) in Form eines der folgenden Artikel ausgebildet ist:
- ein Lebensmittelbehälter (13),
- ein Sitz (11),
- ein Teil eines Sitzes (11),
- ein Teil eines Bettes,
- eine Prothese,
- ein Schuh (17),
- ein Kulturbehälter (14).

11. Vorrichtung nach Anspruch 10, wobei es sich bei dem Stimulationsgeber (30) um eine Flasche (13) handelt.

## Claims

1. An energy stimulation device (10) including at least one stimulation generator (20) provided with at least one first metal body (24) and at least one second metal body (28) in electrical and mechanical contact with the first metal body, the first metal body and the second metal body comprising different metals,
the device further includes at least one stimulation transmitter (30), the stimulation transmitter comprising a rigid support (32) which is distinct from the stimulation generator, and the stimulation generator (20) being fastened on the rigid support (30), the rigid support not being flexible manually without tools,
**characterised in that**
the stimulation generator (20) is in the form of a flat plate with a substantially planar main face (22) facing the rigid support (32).

2. The device according to claim 1, wherein the rigid support (32) of the stimulation transmitter (30) has dimensions which are greater than those of the stimulation generator (20)

3. The device according to any one of claims 1 to 2, wherein the rigid support (32) is made of an electrically insulating material.

4. The device according to claim 3, wherein the rigid support (32) of the stimulation transmitter (30) is made of a material selected from: glass, stone, wood, a mineral and a ceramic

5. The device according to any one of the preceding claims, wherein the stimulation transmitter (30) is a rock crystal, the rock crystal constituting said rigid support (32).

6. The device according to any one of the preceding claims, wherein the first metal body (24) is made of copper and the second metal body (26) is made of zinc.

7. The device according to any one of the preceding claims, wherein the first metal body (24) and the second metal body (26) are superimposed pads, having main faces which are in contact.

8. The device according to any one of claims 1 to 6, wherein the first metal body (24) and the second metal body (26) are concentric.

9. The device according to any one of the preceding claims, wherein the stimulation transmitter (30) has at least one of a polished planar face (33) and a portion forming a tip (31).

10. The device according to any one of the preceding claims, wherein the stimulation transmitter (30) is configured as one of the following items:
- a food container (13),
- a seat (11),
- a seat portion (11),
- a bed portion,
- a prosthesis,
- a shoe (17),
- a cultivation container (14).

11. The device according to claim 10, wherein the stimulation transmitter (30) is a bottle (13).
